(19) Europäisches Patentamt / European Patent Office / Office européen des brevets

(11) **EP 3 338 082 B1**

(12) **EUROPEAN PATENT SPECIFICATION**

(45) Date of publication and mention of the grant of the patent:
**10.04.2024 Bulletin 2024/15**

(21) Application number: **15753034.6**

(22) Date of filing: **19.08.2015**

(51) International Patent Classification (IPC):
**G01N 29/06** (2006.01)  **G01N 29/44** (2006.01)
**G01N 29/30** (2006.01)  **A61B 8/00** (2006.01)
**A61B 8/08** (2006.01)  **G01S 7/52** (2006.01)
**G01S 15/89** (2006.01)

(52) Cooperative Patent Classification (CPC):
**G01N 29/0654; A61B 8/5207; A61B 8/5253;
G01N 29/069; G01N 29/30; G01N 29/4418;
G01N 29/4436; G01N 29/4463; G01N 29/4472;
G01S 7/5202; G01S 15/8977**

(86) International application number:
**PCT/EP2015/069088**

(87) International publication number:
**WO 2017/028920 (23.02.2017 Gazette 2017/08)**

(54) **ULTRASONIC MEASUREMENTS FOR RECONSTRUCTING AN IMAGE OF AN OBJECT**

ULTRASCHALLMESSUNGEN ZUR REKONSTRUKTION EINES BILDES EINES OBJEKTS

MESURES ULTRASONORES POUR RECONSTRUIRE UNE IMAGE D'UN OBJET

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**27.06.2018 Bulletin 2018/26**

(73) Proprietors:
- **Fraunhofer-Gesellschaft zur Förderung
  der angewandten Forschung e.V.
  80686 München (DE)**
- **Technische Universität Ilmenau
  98693 Ilmenau (DE)**

(72) Inventors:
- **DEL GALDO, Giovanni
  98693 Martinroda (DE)**
- **RÖMER, Florian
  98693 Ilmenau (DE)**
- **IHLOW, Alexander
  98693 Ilmenau (DE)**
- **OSMAN, Ahmad
  66121 Saarbrücken (DE)**
- **VALESKE, Bernd
  66802 Überherrn (DE)**
- **HANKE, Randolf
  90617 Puschendorf (DE)**
- **BÄHR, Werner
  66271 Kleinblittersdorf (DE)**

(74) Representative: **Pfitzner, Hannes et al
Schoppe, Zimmermann, Stöckeler
Zinkler, Schenk & Partner mbB
Patentanwälte
Radlkoferstraße 2
81373 München (DE)**

(56) References cited:
WO-A1-2015/140177    DE-A1- 3 321 269
GB-A- 2 192 120      KR-A- 20080 102 785
US-A- 5 817 023      US-A1- 2010 064 811
US-A1- 2013 253 325  US-A1- 2015 078 125

EP 3 338 082 B1

**Description**

Embodiments disclosed herein refer to applications of compressed sensing theory to the problem of material diagnostics using ultrasonic measurements. Embodiments of the present invention refer to a method for reconstructing an image of an object based on received signals and prior knowledge signals and to a corresponding apparatus.

The field of this invention is ultrasound imaging incorporating compressive sensing and a-priori information with possible application in the field of, e.g., non-destructive testing and structural health condition monitoring.

Several techniques exist to acquire ultrasound measurement data, such as conventional ultrasonic transducers, phased array transducers, or the sampling phased array. The sampling phased array (SPA) is described in the patent of Kröning et al. [10]. The method uses ultrasound for the non-destructive examination of a test body, where a number of $n$ ultrasonic transducers are disposed on the surface of a test body (in an unordered or ordered manner). These transducers may be used as transmitters or receivers. For transmitting, $i \geq 1$ transducers are activated. For reception, a number of m transducers is used ($i \leq m \leq n$).

Using an array of transducers enables focusing of the excitation signal. The aim of focusing is to concentrate the ultrasonic energy at a single point, or towards a single direction, or within a single confined volume. The sampling phased array (SPA) shifts the focusing step to the post-processing of the signals, i.e., from the data recorded by the transducers a focused signal at any point, direction or area can be computed.

In general, the intention is to produce an image of the object which represents physical properties of the object, such as the local absorption coefficient or a boundary between regions with different impedances. By focusing, either physically realized or computed in post-processing, it is possible to inspect (i.e., gather information) from a single point, or direction or confined area within the object and/or volume.

A comparison with a reference might be also of interest: The patent of Kruger et al. [11] describes a method and system for determining material properties using ultrasonic attenuation. It works for any ultrasonic technique (e.g., piezoelectric transducers, laser-ultrasonics and EMATs), any type of wave (e.g., longitudinal, shear and surface waves), and any generation/detection configuration (pulse echo, through-transmission, and pitch-catch). As material properties produce frequency dependent attenuation, the spectra of ultrasonic interaction signals from an object are compared to the spectrum of a reference ultrasonic signal from a reference part.

Compressive sensing (CS) [5, 6, 7] allows the reconstruction of a sparse signal from a linear combination of a small number of measurements by an optimization process. Thereby, signal sparsity can be exploited in various ways: The patent of Eldar et al. [8] describes methods and apparatuses for sub-Nyquist sampling of short pulses. An analog signal, which consists of a sequence of pulses confined to a finite time interval is sampled at a sampling rate that is lower than the Nyquist rate. This is done by computing sampled values based on Gabor coefficients with respect to a predefined windowing function. Various variants are claimed for linear combinations of Gabor coefficients and superpositions of the analog signal or parts of the analog signal. The application of the methods to ultrasound signals is explicitly claimed (claim 5). More details are found in, e.g., Innovation Rate Sampling of Pulse Streams With Application to Ultrasound Imaging [16] and Xampling in Ultrasound imaging [17].

The patent of Bao et al. [3] describes an ultrasonic signal reestablishing method based on sparse data where the computational complexity of Othogonal Matching Pursuit (OMP) is decreased by the use of an Artificial Fish Swarm Algorithm. This is shown using a CS-framework with a Gabor dictionary and random measurement matrices (Bernoulli/Gaussian distributions) for simulations.

CS in ultrasound has been discussed in various ways for medical applications. Compressive sensing in medical ultrasound [12] suggests to exploit sparsity in the distribution of scatterers, sparsity of the raw channel data in some basis (e.g., wave atoms), sparsity in the 2D Fourier transform of bandlimited RF signals, or sparsity of the Fourier transform of the Doppler signal.

Apart from medical applications, CS in ultrasound is currently an emerging field in nondestructive testing. The main drawback in ultrasonic NDT is the resolution in echo separation. The common approach to increase the resolution is an increased frequency. However, a higher frequency leads to a lower penetration depth. A better approach is to exploit sparsity in some domain by using sparse recovery techniques.

Azimipanah [1, 2] shows that the CS approach can be considered in ultrasonic NDT. The signal is assumed to be sparse in frequency domain. Three algorithms (Incoherent CS, Multiple Measurement Vectors-CS, CS in Synthetic aperture imaging (SAI)) are introduced and compared to standard techniques (TFM, MUSIC, MVDR). The compression was achieved by either using less frequency bins or less array sensors.

The works of Zhang, Harvey, and Braden [18, 19, 20] focus on the evaluation of microelectronic packages, especially their solder bonds. Microelectronic packages consist of several layers that are relatively thin, so high resolution is required to be able to detect flaws. They call their technique SSR-AMI or learning overcomplete representation based AMI (LORAMI). They exploit sparsity in the phase plane to separate echoes using a frequency of 50 MHz. An overview on several sparse signal recovery techniques and its application in NDT can be found in [21].

NDT using lamb waves (which perform well in e.g. metallic structures) is found in the works of Kexel et al. [9] and Perelli et al. [13]. In [9] the time-domain signal is considered to contain only a small number of relevant pulses and is therefore sparse. In [13] CS is implemented using the Warped Frequency Transform (WFT), which has a Fourier basis.

Soussen et al. [15] and Bossmann et al. [4] use sparse recovery for blind deconvolution. Their work is based on the assumption that the reflectivity function has a sparse distribution, i.e., the number of scatterers is small.

Ricci et al. [14] combine chirp signals and sparse recovery to improve air-coupled ultrasonic NDT.

The US 2010/064811 A1 refers to a method for imaging ultrasonic inspections, wherein the ultrasonic excitation signal is emitted in a cone-shaped manner. The US 2013/253325 A1 describes a method for improving the quality of ultrasonic images,

The WO 2015/140177 A describes an apparatus which can be used in context of image reconstruction. The GB 2192120 A describes a concept for reconstructing objects for limited angle scannings in computerized tomography. The KR 20080102785 describes a method for finding location or 3D integral image reconstruction and apparatus thereof. Furthermore, the DE 3321269 A1 should be mentioned.

Since all of the above discussed approaches suffer from some drawbacks, it is a common goal to improve the above concepts.

An objective of a the- first aspect is to improve the signal measurement or, preferably, the series of measurements with respect to the generated excitation signals in order to achieve a better reconstruction.

The first aspect provides a method for providing multiple (equally informative) measurements of an object. The method comprises the steps of providing a plurality of non-focused excitation signals to a volume comprising the object in accordance with excitation properties and receiving a plurality of response signals resulting from reflections of the non-focused excitation signals at the object and/or within the volume. Furthermore, the method comprises the step of reconstructing an image of the object based on the received signals. Here, the providing of the plurality of non-focused signals comprises the sub-step of generating ultrasonic waves such that the ultrasonic energy is not in a single point or towards a single direction or within a confined volume within the object and/or volume.

This aspect 1 regarding the non-focusing ultrasonic measurements is based on the findings that non-concentrated ultrasonic waves within a volume surrounding the object or within the object itself enables improvements regarding the reconstruction of the image of the object because systematical effects of the measurement caused due to focused excitation signals are avoided. For example, the scanning depth is a function of the frequency and the excitation energy. By varying these two excitation parameters the scanning may be performed within different depths. By doing so, the object and the surround is scanned within higher and lower levels, wherein variations of the quality and the resolution are avoidable. Preferably, the scanning is performed using multiple equally informative measurements (scans).

According to further embodiments, the excitation parameters (cf. above) may be adapted based on the reconstruction process or, in more detail, based on the result of the reconstruction. For example, the excitation frequency and the excitation magnitude for the next measurement may be adapted based on an additional step of analyzing the reconstructed image. Another example would be to use focused signals generated using phased array technique in order to focus on a specific depth where more details are required for the reconstruction at the corresponding location.

A further embodiment provides an apparatus for performing multiple (equally informative) measurements of an object. The apparatus comprises an interface for providing the plurality of non-focused excitation signals, an interface for receiving the plurality of response signals and a processor for reconstructing the image of the object based on the received signals. As discussed with respect to the method, the apparatus provides the plurality of non-focused signals by generating ultrasonic waves such that ultrasonic energy is not concentrated in a single point or towards a single direction or within a confined volume with the the object and/or in the volume.

An objective of a second aspect is to improve the reconstructing process regarding its performance.

This objective is solved by the independent claims 1 and 7.

The invention provides a method for reconstructing an image of an object based on received signals and prior knowledge signals. The method comprises the steps of removing information within the received signals that are contained by the received signals and the prior knowledge signals to obtain a residual signal and reconstructing an image based on the residual signal.

Teachings according to this 2nd aspect are based on the findings that a plurality of information/signals does not have to be reconstructed because this information or portions of the image are still available from a reconstructed reference object or from previous measurements. Therefore, the information available is reduced within a first step such that only a residual signal is used for the reconstruction, wherein the portions removed within the first step are added afterwards in order to complete the image after being reconstructed.

According to the invention the reconstruction process is based on the following formula:

$$\arg \min_{\Delta f} g(\Delta X, \text{ForwardModel}(A, \Delta f)) + \lambda \cdot h(\Delta f),$$

wherein Forward Model (*A,Δf)* is a function that simulates the measured signal we would obtain if we excited a volume described by the image $\Delta f$ with the excitation signal described by $A$, the function $g(X_1, X_2) \in \mathbb{R}$ quantifies the difference between the two measured signals $X_1$ and $X_2$, and the function $h(\Delta f) \in \mathbb{R}^+$ quantifies the sparsity of the residual image $\Delta f$.

According to the invention, the so-called prior knowledge, i.e., the information on the image available before the measurement, comprises signals belonging to one or more previous measurements of a reference object, from signals belonging to one or more measurements of the object to be reconstructed and serving as reference, from one or more images of a reference object, from one or more images of a set of reference objects and/or from one or more simulated images of a reference object.

According to further embodiments, there are two different methods according to which the reconstructing is performed in case of a misalignment between the reference object and the object to be reconstructed.

According to a first method the basic method comprising the two steps of removing and reconstructing is enhanced by the step of synthesizing a virtual reference object based on a reference object misaligned with the object to be reconstructed before removing the information, wherein the synthesizing is performed taking into account the misalignment. According to a second method, the basis method comprising the steps of removing and reconstructing is enhanced by the step of aligning the reference object misaligned with the object to be reconstructed to obtain the difference between the misaligned reference object and an aligned reference object and performing the step of reconstructing the image based on the residual signal and the difference between the misaligned reference object and the aligned reference object. Alternatively, this second method may be described by the following formula:

$$\arg \min_{\Delta f} g(\Delta X, \text{ForwardModel}(A, \Delta f + f_{\text{mis}})) + \lambda \cdot h(\Delta f),$$

wherein ForwardModel $(A, \Delta f)$ is a function that simulates the measured signal we would obtain, if we excited a volume described by the image $\Delta f$ with the excitation signal described by A, the function $g(X_1, X_2) \in \mathbb{R}$ quantifies the difference between the two measured signals $X_1$ and $X_2$, and the function $h(\Delta f) \in \mathbb{R}^+$ quantifies the sparsity of the residual image $\Delta f$, and wherein the signal $f_{mis}$ is additionally contained in the data fidelity term to correct difference between the misaligned reference object and the aligned reference object.

According to a further embodiment an apparatus is provided which is configured to reconstruct an image of an object based on received signals and prior knowledge signals. The apparatus comprises a processor configured to remove information within the received signals that are contained by the received signals and the prior knowledge signals to obtain a residual signal and configured to reconstruct the image based on the residual signal.

An objective of another aspect (aspect 3) is to increase the performance time of the entire measurement process including the measurement itself and the reconstruction.

This objective is solved by the subject matter according to the independent claims 16 and 22.

An embodiment provides a method for preforming multiple measurements of an object to be reconstructed. The method comprises the steps of performing a first measurement comprising the step of providing a plurality of excitation signals to a volume comprising the object and the step of receiving a plurality of response signals resulting from reflections of the excitation signal at the object and/or within the volume.

The method further comprises the step of starting to reconstruct an image of the object based on the received signals of the first measurement. Furthermore, the method comprises the step of performing a second measurement comprising the step of providing the plurality of excitation signals to the volume and the step of receiving a plurality of response signals resulting from reflections of the excitation signals at the object and/or within the volume. The last step of this basic method is continuing to reconstruct the image of the object based on the received signals of the first and second measurement, wherein the step of starting to reconstruct is in parallel to the step of performing the second measurement.

Embodiments of this third aspect are based on the principle that it has been found out that the reconstruction may be sped up by performing the measurement and the reconstruction in parallel. Starting to reconstruct the object is possible by the point of time when the first measurement data, e.g., after the first measurement, is available. Therefore, the reconstruction is performed directly after finishing the first measurement, wherein the reconstruction is updated every time when new measurement data is available.

According to further embodiments, the reconstruction is finalized with the usage of the last data obtained from the last measurement.

According to further embodiments the three above discussed methods according to the Aspects 1 to 3 are combinable, such that two methods are carried out together or the three methods are carried out together.

In particular, it should be emphasized that the method for performing multiple measurements of the object (Aspect 1) and the method for performing multiple measurements of an object to be reconstructed (Aspect 3) can be combined. The optional feature in connection with the adaption of the excitation properties is also applicable. From this point of view, this means that the method according to Aspect 1 belongs to a first portion of the measurement chain, wherein the method according to the Aspect 3 belongs to a successive portion of the measurement chain. Furthermore, the method according to Aspect 2 is combinable with the two previously discussed methods. Nevertheless, it should be noted that all three methods may be performed using a computer or another device comprising a processor such as a CPU. Therefore, further embodiments refer to a computer program comprising a program code, in accordance with which, when running on a computer, the method steps according to the methods of Aspects 1 to 3 are performed.

Additional and optional features are defined by the dependent claims.

Embodiments will be discussed below referring to the enclosed drawings, wherein:

Fig. 1a          shows a full chart of a method according to the first aspect;

Fig. 1b          shows a block diagram of an apparatus according to the first aspect;

Fig. 1c          shows a block diagram of an enhanced apparatus according to the first aspect;

Fig. 2a          shows a flow chart of the method according to the second aspect;

Fig. 2b          shows a block diagram of an apparatus according to the second aspect;

Figs. 2c to 2f   show block diagrams of enhanced apparatuses according to the second aspect;

Fig. 2g          illustrates an optional feature of the method and apparatus according to the second aspect;

Fig. 3a          shows a flow chart of a method according to the third aspect;

Fig. 3b          shows a block diagram of an apparatus according to the third aspect;

Fig. 3c          illustrates the background of the third aspect;

Fig. 4a          shows a block diagram of an apparatus for connecting a PC to an ultrasonic head according to a conventional approach; and

Fig. 4b          shows a block diagram of an apparatus according to the fourth aspect.

Embodiments of the present invention will subsequently be discussed below referring to the figures, wherein the same reference numerals are provided to objects having the same or a similar function. Therefore, the description of the blocks having the same reference numeral are interchangeable or mutually applicable.

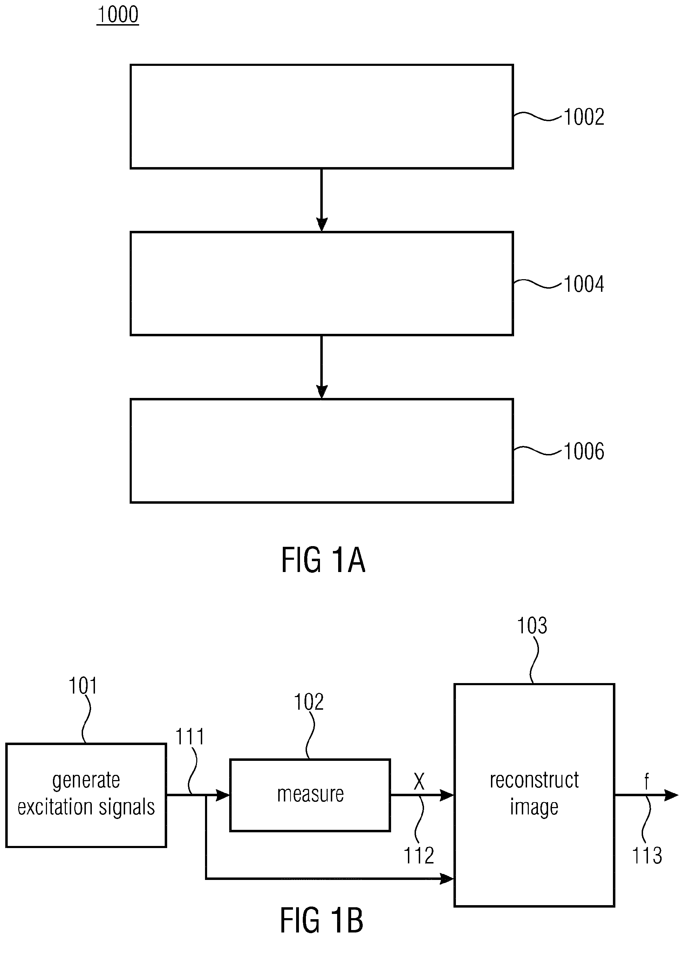

Fig. 1a shows a block diagram of the method 1000 for performing multiple (preferably equally informative) measurements of an object such as ultrasonic measurements, e.g., an organ in the human body in case of medical applications or a fault in a material in case of industrial applications (material/quality testing). The method 1000 comprises the three basic steps 1002, 1004 and 1006. The first step 1002 is the providing of a plurality of non-focused excitation signals to a volume comprising the object in accordance with excitation properties. The second step 1004 is receiving a plurality of response signals resulting from reflections of the non-focused excitation signals at an object and/or in the volume. The received signals may be referred to by the letter x. Based on the received signals x, the reconstruction is performed in a third step 1006.

The beneficial features are mainly integrated into the step 1002 of providing the plurality of non-focused signals. Non-focused means that the signals, i.e., the generated ultrasonic signals, are output using an ultrasonic head in a manner such that the ultrasonic energy is not concentrated in a single point or towards a single direction or within a confined volume within the object and/or volume. This generation of the ultrasonic signals may be influenced using the excitation properties, e.g., an excitation frequency, an excitation frequency range, an excitation magnitude and/or an excitation direction. With these properties the signal generator is typically configured which drives the ultrasonic head.

The apparatus used for the above method is illustrated by Fig. 1b.

Fig. 1b shows a signal generator 101 output the signal using an ultrasonic head (not shown) and the measurement unit 102 receiving the reflected signals. This unit 102 may also use the ultrasonic head for receiving the reflected signals. Based on the received signals x, a processor 103 performs the reconstruction, wherein the processor 103 typically has a second input for the excitation signal output by the signal generator 101 because typical reconstruction algorithms use this signal for the reconstruction process.

The functionality of the apparatus shown by Fig. 1b will be discussed below.

Block 101, the signal generator, generates excitation signals either as

- weighted pulses
- noise-like signals

such that the ultrasonic energy does not concentrate in a single point or towards a single direction or within a confined volume within the object and/or volume.

Examples for such signals are: pulses having random i.i.d. Gaussian amplitudes placed at random delays, or signals so constructed such that only one or a subset K up to N of the elementary elements are being excited at the same time and frequency (1, K or N elements out of N elements). As a result, the ultrasonic energy will be distributed fairly uniformly within the object and/or volume, or, if treated as a stochastic process, the expected value of the energy will be fairly constant within the object and/or volume, meaning that in one specific realization, the ultrasonic energy might concentrate in two or more points or towards two or more directions or in two or more confined volumes.

Block 102 represents the measurement process, namely the signals 111 are reproduced by the K transmit transducers, while the signals 112 are measured by the N receive transducers.

Block 103 contains the reconstruction algorithm which takes the measurements 111 to recover the image 113.

The usage of the above described non-focused signals are advantageous because in fairly homogeneous materials, the speed of sound may be considered to be constant and the material properties are the same in all directions. Consequently, the wave fronts of elementary waves generated via non-focused signals are spherical and propagate in perpendicular direction to the wave front and spread through the homogeneous medium/volume without focusing at a specific depth or location. This gives equally informative received signals independendly of a specific depth or orientation.

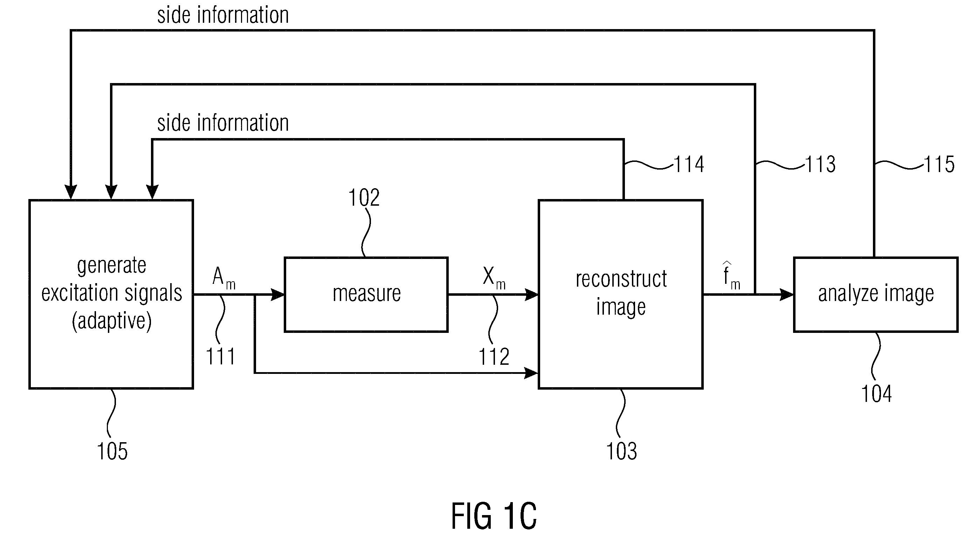

According to further embodiments, the above discussed excitation properties may be adapted using an adaption mechanism which will be illustrated with respect to Fig. 1c. The adaption mechanism provides means to improve the reconstructed image by means of adapting the excitation signals to the previously computed reconstructed image and/or controlling the number of measurement runs to be carried out.

In this case, M > 1 measurement runs may be carried out. Block 101 from Figure 1b is replaced by block 105. In the first iteration, block 105 generates the excitation signals as 101 would do. However, once the first measurement is available, namely $X_1$, block 103 attempts a first reconstruction, outputting a first reconstructed image $\hat{f}_1$. Optionally, the first reconstructed image is further analyzed by block 104, e.g., in the attempt to estimate defects or other features. Information is now fed back to block 105. This information may be

- 114: side information from the reconstruction algorithm which expresses the quality/reliability of the reconstruction, such as the decay of the residual with the iterations, or the magnitude of the residual after the last iteration

- 113: the reconstructed image itself

- 115: side information from block 104 which expresses the regions of particular interest in the image, or the regions which are affected by larger uncertainty.

Block 105 receives this information and may adapt the next measurement run in one of the following ways:

1. decide to carry out a new measurement with the same excitation signals 111, namely

$$A_m = A_{m-1}$$

2. decide to carry out a new measurement with new randomly drawn excitation signals 111

3. decide to carry out a new measurement with excitation signals designed according to the information obtained from the subsequent blocks, namely 114, 115 or 113.

Solution 1 merely improves the SNR of the measurements. In fact, in this case, at the m-th measurement run, block 103 performs a reconstruction on $\overline{X}_m$ such that

$$\overline{\mathbf{X}}_m = \frac{1}{m}\sum_{i=1}^{m}\mathbf{X}_i \ .$$

Solution 2 leads to a better reconstruction compared to Solution 1, at the expense of a higher computational complexity. In this case, at the m-th measurement run, block 103 may perform a reconstruction on $\overline{\mathbf{X}}_m$ such that

$$\overline{\mathbf{X}}_m = \left[\mathbf{X}_1, \mathbf{X}_2 ... \mathbf{X}_m\right].$$

The reconstruction strategy for Solution 3 is the same as for Solution 2. However, in Solution 3, the excitation signals are designed in such a way that the new measurement is more informative in the certain regions, based on the information in 114, 115, or 113, which may, e.g., identify regions of particular interest or regions that are affected by a larger uncertainty. In so doing, Solution 3 leads to the best reconstruction compared to Solutions 1 and 2.

To provide a concrete example how the adaptation mechanism in Solution 3 may be implemented, let Forward-Model(A, f) be a function that simulates the measured signal (X) we would obtain if we excited a volume described by the image $f$ with the excitation signal described by $A$. Now consider one embodiment where this forward model can be described by a linear operator such that ForwardModel(A, f) = $\Phi(A) \cdot$ f where $\Phi$ (A) is a matrix containing coefficients that depend on the excitation signals $A$. In this case, each pixel/voxel of the volume, represented by one element of the vector f corresponds to one particular column of $\Phi$. Therefore, a set of regions in the volume corresponds to a subset of columns in the matrix $\Phi$. Since the number of voxels is typically significantly larger than the number of measurements, a typical instance of $\Phi$ is highly overcomplete which means that it has significantly more columns than rows. Therefore, columns can be highly correlated which negatively impacts the reconstruction performance since any set of highly correlated columns leads to very similar measurements that are easily confused with each other during the reconstruction. Referring now to the adaptation mechanism used in Solution 3, the excitation signals A can be chosen such that the subsets of columns that correspond to the regions identified via the feedback information in 113, 114, or 115 have a particularly low correlation with each other. For example, a large set of realizations of A can be drawn randomly and the one realization leading to the lowest correlation among the columns in the subsets corresponding to the set of identified regions is chosen for the next measurement.

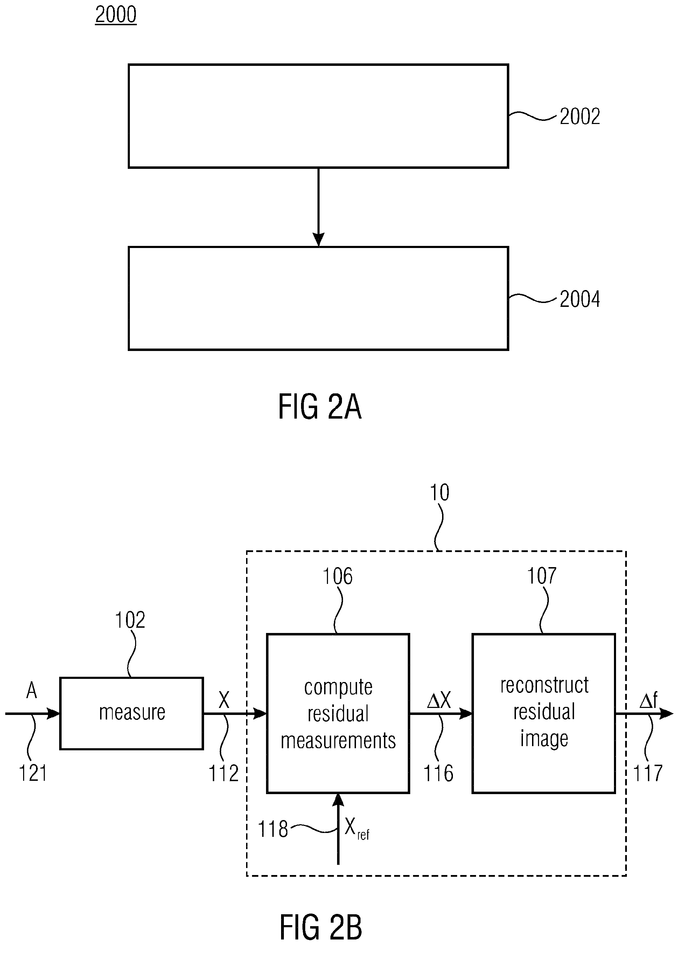

With respect to Fig. 2a a further method 2000 will be discussed. The method 2000 serves the purpose of reconstructing an image of an object based on received signals and prior knowledge signals. The method 2000 comprises the two basic steps 2002 and 2004. The step 2002 is the removing of the information within the received signals that are contained by received signals and the prior knowledge to obtain a residual signal. The step 2004 is reconstructing an image of the object based on the residual signal.

The first step 2002 starts from the measured signal x (cf. Fig. 1b), wherein it should be noted that the measurement of the received signal may also be performed using conventional measurement procedures. This signal x comprises a plurality of signals which are out of interest since they are also available within signals belonging to one or more measurements of a reference object or signals belonging to one or more measurements of the object to be reconstructed and serving as measurement object (e.g., previous measurements) within reconstructed images of the reference object or previously reconstructed objects or within simulated signals. From another point of view, this means that the signals according to the prior knowledge do not add additional information to the measurement. Therefore, this signal available twice may be removed from the measurement in order to relieve the reconstruction process 2004.

This method 2002 will be discussed in detail with respect to the apparatus performing the method 2000 shown by Fig. 2b.

Fig. 2b shows the measurement unit 102 e.g., the ultrasonic equipment receiving the reflected signals and the unit 10 comprising the two units responsible for computer residual measurements by removing the already known signals ($x_{\mathrm{ref}}$) and the unit 107 responsible for reconstructing the image based on the residual signal.

The functionality of the apparatus of Fig. 2b will be discussed below.

Block 106 extracts the residual measurement data $\Delta X$ (116) by comparing the measured data X (112) with the reference measurement data $X_{\mathrm{ref}}$ (118) with the goal to remove the information from the measured data 112 that is already contained in the reference data 118. In one embodiment, this step may perform the difference between 118 and 112, i.e., $\Delta X = X - X_{\mathrm{ref}}$.

Block 107 reconstructs the residual image $\Delta f$ (117) from the residual measurement data 116. Note that in the case that 106 is implemented by taking a difference, 117 can be interpreted as the difference between the desired image of the currently measured object and the reference image. The operation of block 107 can be described by a method to solve the optimization problem given by

$$\arg \min_{\Delta f} g(\Delta X, \text{ForwardModel}(A,\Delta f)) + \lambda \cdot h(\Delta f), \qquad (1)$$

where ForwardModel($A,\Delta f$) is a function that simulates the measured signal we would obtain if we excited a volume described by the image $\Delta f$ with the excitation signal described by A, the function $g(X_1,X_2) \in \mathbb{R}^+$ quantifies the difference between the two measured signals $X_1$ and $X_2$, and the function $h(\Delta f) \in \mathbb{R}^+$ quantifies the sparsity of the residual image $\Delta f$.

For example, we can choose $g(X_1,X_2) = \| X_1 - X_2 \|_2$ and $h(\Delta f) = \|\Delta f\|_1$. In this case, (1) represents a regularized least squares problem which can be solved by a person skilled in numerical optimization. In particular, the optimization problem (1) can be solved by iterative methods such as a projected gradient descent method.

The main advantage of 107 compared to a traditional reconstruction step is that due to the removal of the known prior information, the residual image is expected to contain a significantly reduced amount of information, e.g., a sparse vector with only very few non zero elements. This leads to a faster and more accurate reconstruction of the residual image.

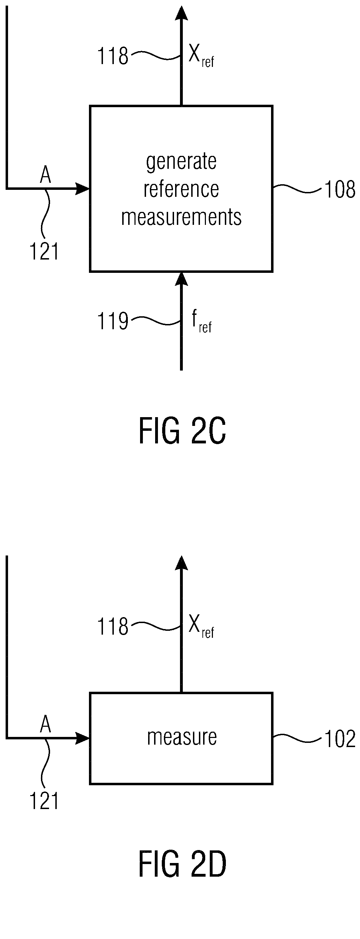

The reference measurement data $X_{\text{ref}}$ (118) can be obtained in two fundamentally different ways depending on the type of prior knowledge that is available. In the case that the prior knowledge is available in form of a reference image (which can be e.g., an image generated from CAD data or a reconstructed image from the measurement of a reference object or a reconstructed image from a measurement of the same object at an earlier time), this reference image is transformed into a set of reference measurements as shown on the left-hand side of Figure 2c. Block 108 takes the reference image and a set of excitation signals 121 and outputs 118 which corresponds to measurement data we would have obtained by measuring an object, which represents the reference image, e.g., a simulation of the forward model. On the other hand, the reference measurement data can also be obtained by an actual measurement of a physically existing object which can be, e.g., a reference object or the same object as the test object measured at an earlier time. This is depicted on the right-hand side of Figs. 2c, d. Fig. 2c represents a first option according to which the reference measurements $X_{\text{ref}}$ from reference image $f_{\text{ref}}$ are generated. Fig. 2d represents a second option according to which the reference measurements $X_{\text{ref}}$ by measuring the physical object, which can be a reference object or the same object at an earlier time, are generated. Note that for both Figure 2c and Figure 2b, the excitation signal 121 may be either the output of 101 (e.g., 121 = 111) or a state-of-the-art excitation signal.

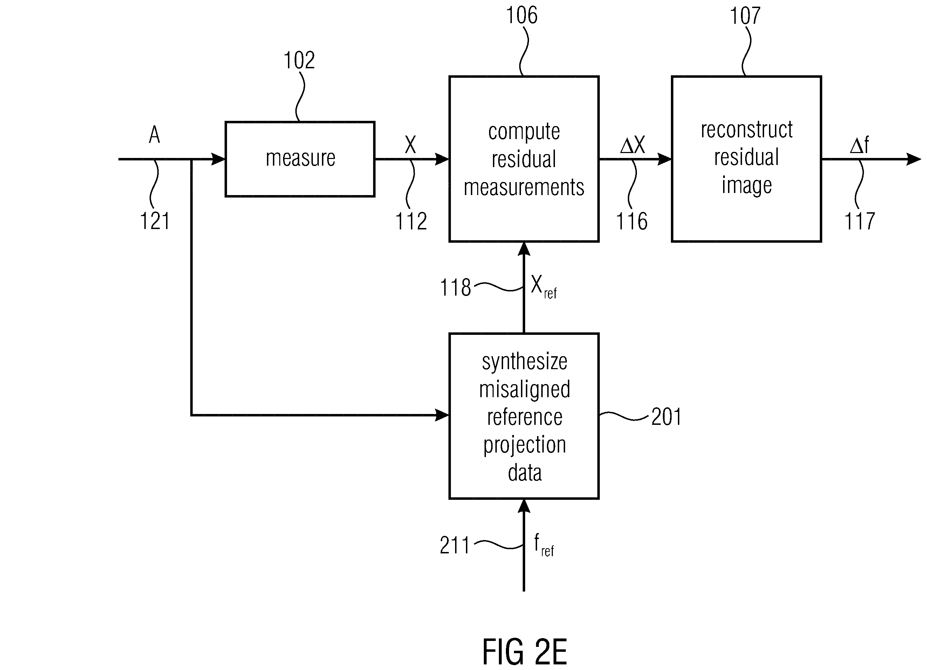

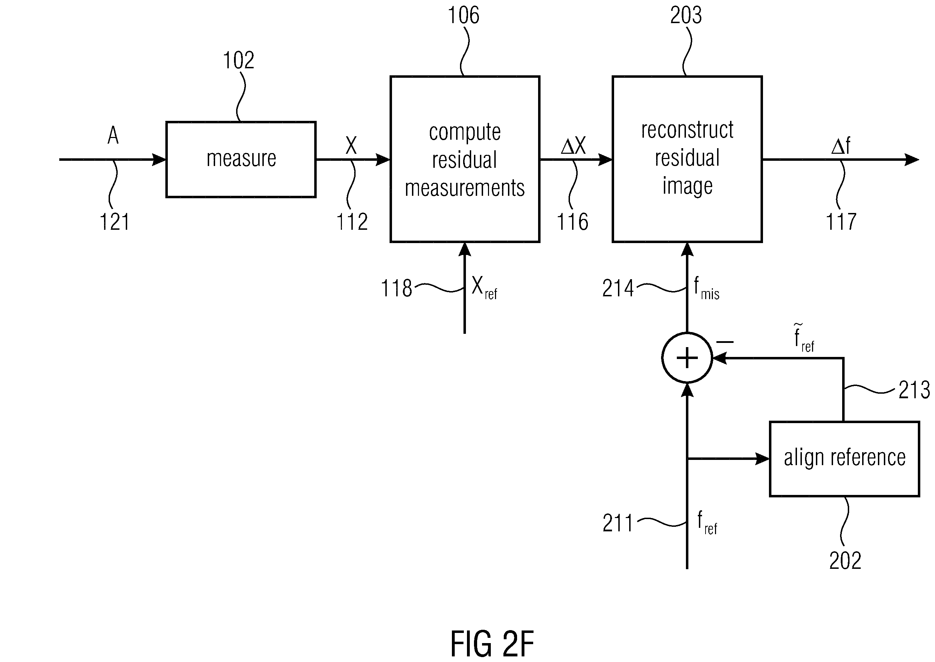

Referring now to Figs. 2e and 2f, an optional extension of the second aspect is described.

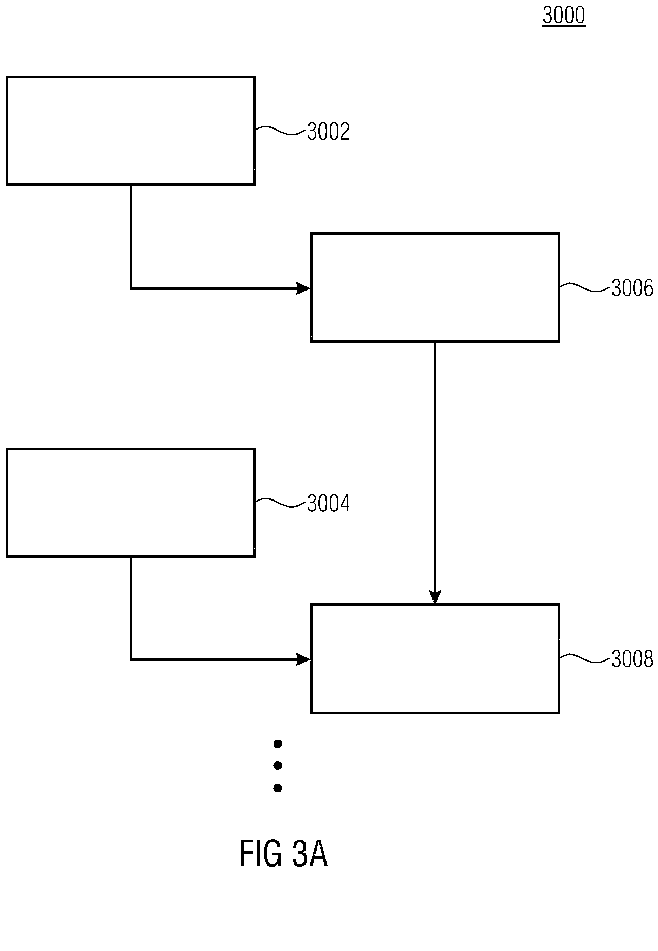

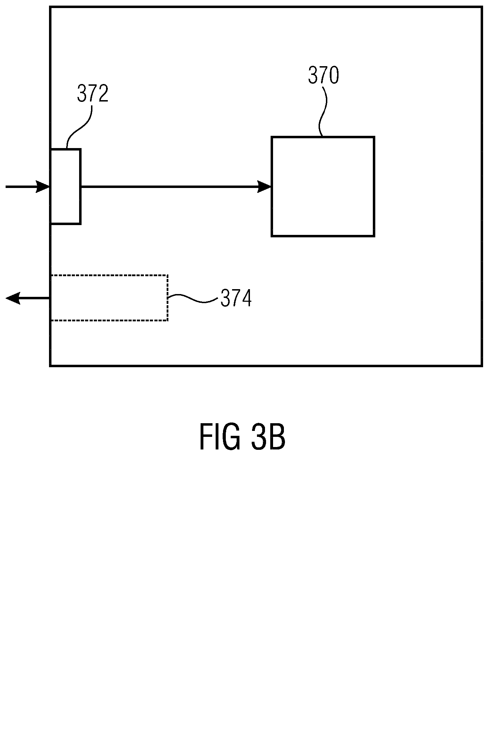

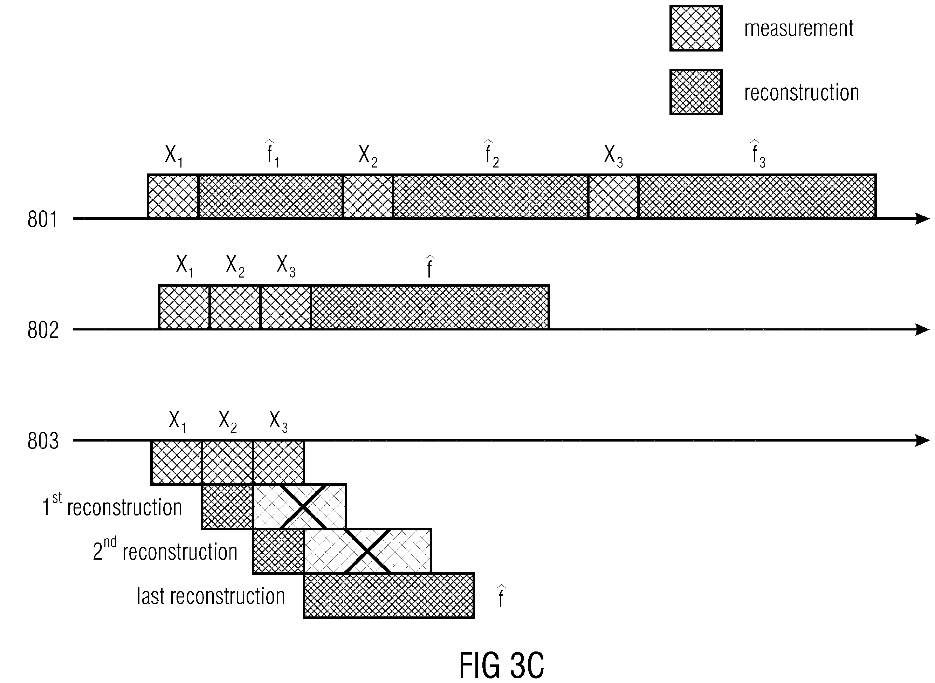

In scenarios where the current test object is not aligned to the object data contained in the a priori known reference image, the reconstruction algorithm described in Figure 3 may not appropriately recover the residual image. For this case, we propose a second approach which explicitly addresses this issue by taking into account the misalignment between the current test object and the reference image. The main difference to the first approach is that the second approach assumes additional information about the objects misalignment to be available.

There are two methods to use the misalignment information for the reconstruction. The first method is shown in Figure 2e. There is an additional block 201 which synthesizes a set of reference measurements 118 from the known reference image 211 by taking into account the misalignment. In other words, 201 generates the measurements we would have obtained if the reference object had been oriented in the same way as the current test object is oriented. Thereby, the reference measurements 118 become more similar to the measured data 112, allowing the block 106 to remove more known information from 112.

The second method is shown in Figure 2f. The information about the misalignment between the reference and the current test object is used in box 202 to align (e.g. rotate and translate) the reference image 211 with respect to the test object. The modified reference image 213, which is now aligned to the current test object, is then subtracted from the reference image 211 yielding the difference image $f_{mis}$ (216). The difference image 216 is then fed to the reconstruction box 203.

In a second step, the reconstruction of the residual image is performed in 203 using as inputs the residual projection data 116 and the difference image 214. The reconstruction step 203 is a block that solves an optimization problem to find the sparse residual image $\Delta f,$ taking into account the misalignment $f_{mis}$ in the data fidelity term (which measures how well the projection of the reconstructed residual image matches the residual projection data $\Delta X$). In the special case that 106 finds $\Delta X$ via a direct subtraction according to AX = $X_{\text{ref}} - X$, the cost function solved by 203 takes the following form

$$\arg \min_{\Delta f} g(\Delta X, \text{ForwardModel}(A, \Delta f + f_{\text{mis}})) + \lambda \cdot h(\Delta f), \qquad (2)$$

Note that (2) is similar to the reconstruction 107 in Figure 2b which solves (1). However, in (2), the signal $f_{mis}$ is additionally contained in the data fidelity term to correct the misalignment between the current test object and the object data contained in the reference image 211. The cost function (2) can be solved by a person skilled in numerical optimization. In particular, it can be solved by iterative methods such as a gradient descent method.

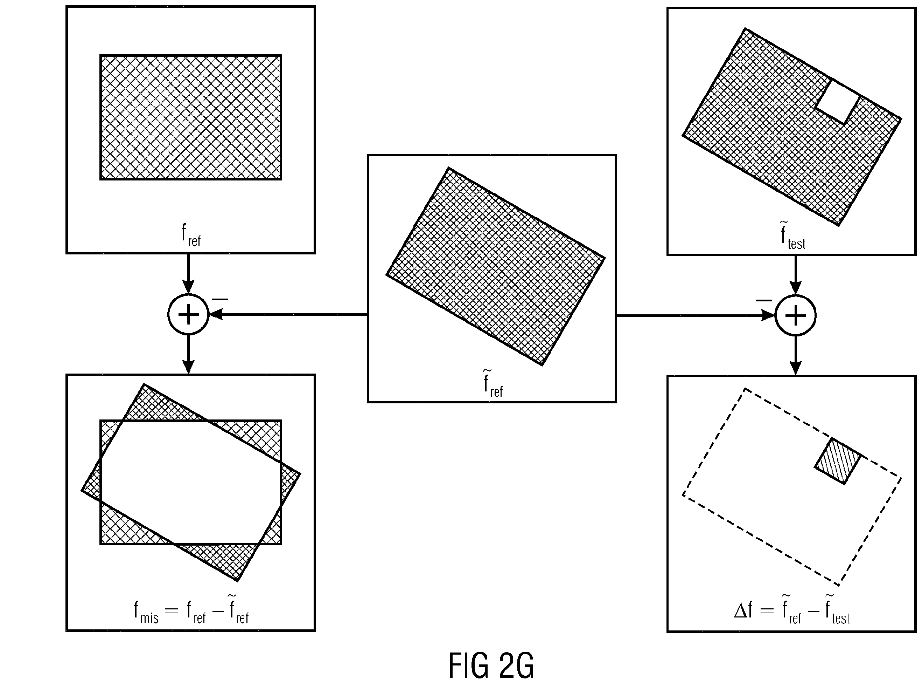

Figure 2g further illustrates the signals $f_{\text{ref}}$, $\tilde{f}_{\text{ref}}$, and $f_{mis}$. The known reference image $f_{\text{ref}}$ is shown in the top-left corner of Figure 2g. We assume that our test object (whose image we call $\tilde{f}_{\text{test}}$, shown in the top-right corner of Figure 2g) is given by a rotated version of the known reference image (which we call $\hat{f}_{\text{ref}}$, shown in the middle of Figure 29) from which the desired residual image $\Delta f$ is subtracted so that $\tilde{f}_{\text{test}} = \hat{f}_{\text{ref}} - \Delta f$. The difference between the reference image $f_{\text{ref}}$ and the misaligned version $\hat{f}_{\text{ref}}$ is called $f_{mis} = f_{\text{ref}} - \hat{f}_{\text{ref}}$. It is shown in the bottom left corner of Figure 2g, where red and green indicate positive and negative values, respectively. In the cost function (2) that is solved by step 203, we optimize over the sparse residual image $\Delta f$, enforcing its sparsity via the term $h(\Delta f)$. At the same time, our residual projections $\Delta X$, which are found from the difference $X_{\text{ref}} - X$ correspond to $f_{\text{ref}} - (\hat{f}_{\text{ref}} - \Delta f) = f_{mis} + \Delta f$ in the image domain. This explains why $f_{mis} + \Delta f$ is used in the data fidelity term in (2).

With respect to Fig. 3a, a method 3000 for performing multiple measurements of an object to be reconstructed will be discussed. The method 3000 comprises two parallel activities, namely the measurement activity and the reconstruction activity. The measurement activity comprises at least a first measurement 3002 and a second measurement 3004. The two measurements are performed subsequently to each other, wherein each measurement comprises the step of providing a plurality of excitation signals to a volume comprising the object and the step of receiving a plurality of response signals resulting from reflections of the excitation signals at the object and/or within the volume.

After finishing the first measurement activity, the first step of the reconstruction activity begins. The first reconstruction step 3006 is starting to reconstruct an image of the object based on the received signals of the first measurement. This reconstruction is performed as long as the second measurement is not finished. After finishing the second measurement, the second step of the reconstruction activity 3008, namely the continuing to reconstruct the image of the object based on the received signals of the first and second measurement, is performed.

The apparatus performing the steps is illustrated by Fig. 3b. The apparatus comprises a CPU 370 configured to perform the reconstruction and an input interface 372 by which the received signals of the first and second measurement are received. Furthermore, the apparatus may comprise a controller 374 controlling the measurements.

The method 3000 performed by the apparatus of Fig. 3b enables to speed up the total time needed to carry out measurements and reconstruction. With respect to Fig. 3c the detailed proceedings will be discussed.

Figure 3c shows several time axes, where blocks of different hatchings represent the measurements and reconstructions. The variables reported above the time slots indicate the output of the corresponding procedures. For instance, after the first measurement the measurement data $X_1$ will be available for further processing. Time axes 801 and 802 depict two straightforward approaches which may be applied when several measurement runs of the same specimen are taken. In 801, after each measurement run, a new reconstruction is carried out. The i-th reconstruction makes use of all available measurements, namely $X_1$, $X_2$, ... $X_i$. If the excitation signals differ, later reconstructions will take more time than earlier ones. In 802, all measurements are carried out sequentially and then a reconstruction is carried out at the end, making use of all the collected data.

Time axis 803 depicts the method proposed in this invention. As soon as one measurement has been carried out, a first reconstruction is started. In parallel to this, a second measurement is carried out. Once the measurement data $X_2$ is available, the first reconstruction is not further pursued and a second reconstruction is started. Only the last reconstruction is brought to convergence, leading to the estimate $\hat{f}$. The i-th reconstruction makes use of the intermediate results of the previous one, i - 1. This is achieved following one of the following methods, depending on how the measurements are carried out:

1. When the excitation signals are the same, $A_i = A_{i-1}$: The cost function is updated with the measurement matrix being equal to the weighted sum of the previous measurements. This is not dissimilar to the process explained in the optional adaptation mechanism in Aspect 1.

2. When the excitation signals differ, $A_i \neq A_{i-1}$: The i-th reconstruction will aim at solving a different optimization problem than the one of the (i-1)-th reconstruction. In fact, the cost function will now contain as measurement the concatenation of the measurements $X_1$, $X_2$. .. $X_i$, not dissimilar to the process explained in the optional adaptation mechanism in Aspect 1. As initial solution to start the optimization, the last estimate from the (i - 1)-th reconstruction is used. This aspect may be combined with Aspect 2 (residual reconstruction).

Another embodiment provides a method and an apparatus to reduce the effort in transmitting signals from and to the ultrasonic head.

Assume K transducers are used to transmit (Tx), N transducers are used to receive (Rx). If the same transducers are used both for Tx and Rx, then $K = N$.

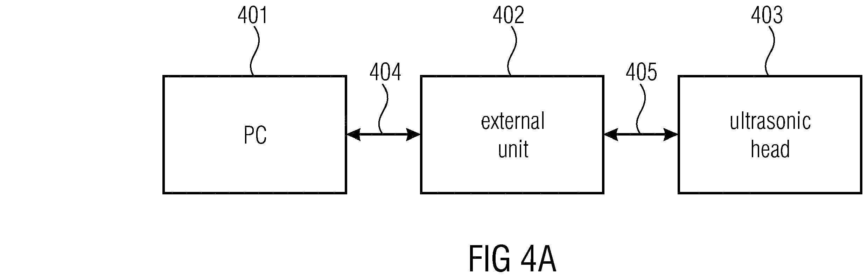

Figure 4a depicts how a traditional setup to carry out ultrasonic measurements. A PC (Block 401) generates the signals which should be played back by the transducers in the ultrasonic head. These signals, in a digital representation, are passed to Block 402 (usually via an Ethernet connection 404), which converts them into analog signals. These are passed to ultrasonic head 403 (usually via coaxial or twisted pair cables).

The signals received by the transducers in 403, after being processed by analog filtering and amplifying are transmitted back to 402, which then converts them into digital signals and passes them back to block 401, where an appropriate software carries out the processing. In more modern test equipment, the analog amplification, analog filtering, ADC conversion and signal shifting and summing (can both be done analog or digital) are being encapsulated within one unit as near as possible to the ultrasound head. Increasing attempts to integrate some of these functionalities within the ultrasound head are actively explored. Moreover, specific signal processing and reconstruction algorithms may be implemented on dedicated graphic cards, thus reducing the computational effort required to be done at CPU level, so that the PC in this case plays the role of interfacing and mediation between the ultrasound system (block 402 and 403) and the user. The PC options include storage, reuse and archiving of test parameters and test results. The communication between the PC and the data processing front end can as well be realized using fast hardware-network protocols, faster than Ethernet, such as the Serial Advanced Technology Attachment (SATA) which offers data transfer up to 6 Gbits/second from the block 402 to the PC.

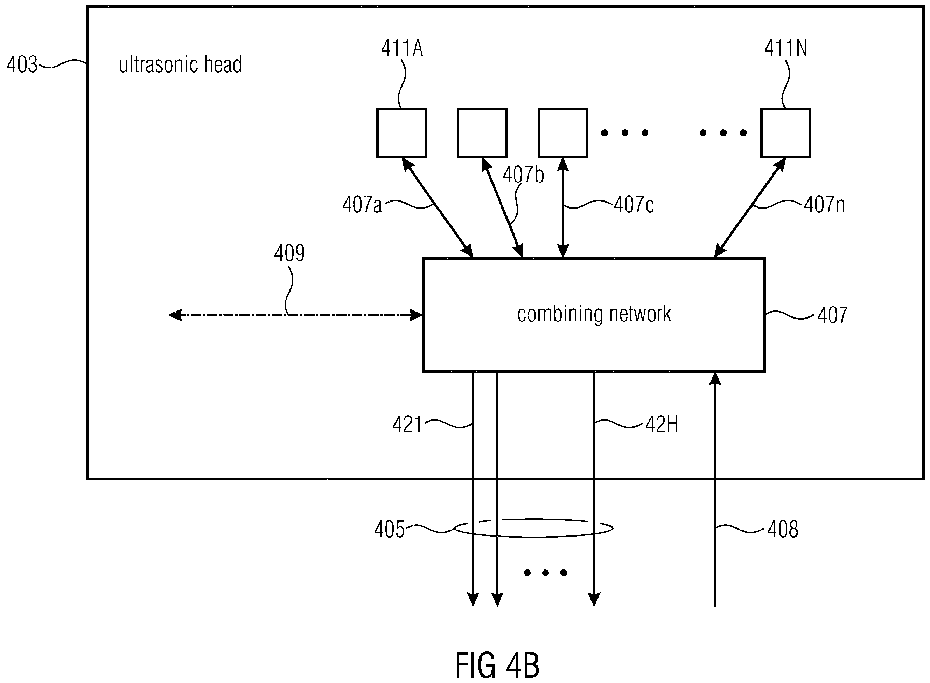

With this arrangement, the connection 405 typically has either N+K analog channels in the case of simplex communication or max(K,N) if duplex communication is used. To reduce the number of necessary channels, an embodiment of this invention features the mechanism shown in Figs. 4b.

The basic configuration of Fig. 4b comprises the combiner 407, the computer interface 405 and the plurality of interfaces 407a, 407b, 407c to 407n for the plurality of ultrasonic heads 411n. The combiner 407 comprises a mixer performing the up and down mixing between the interfaces 407a, 407b, 407c to 407n and the computer interface 405.

In the ultrasonic head, an analog combining network 407 has the task to mix the N signals coming from the N transducers 411 to 41N into H signals 421 to 42H. The combination is driven by the digital control signal carried by 408. In the simplest embodiment, the h-th signal 41h is obtained as a linear combination (e.g., a weighted sum) of the signals 411 to 41N where the weights can be different for each h = 1, 2, ... ,H.

The configuration of the combining network 407 is accessible via the connection 409 which communicates the topology, the description and alterations from and to the combining network.

With respect to the above discussed four embodiments, it should be noted that all embodiments are preferably applied in combination to each other, wherein each embodiment according to one of the Aspects 1 to 4 may be used independently.

For example, the embodiment of Aspect 4 (residual reconstruction with or without optional registration) may of course be used in combination with the measurement procedure of Aspect 1 but also in combination with a conventional measurement procedure. Furthermore, this embodiment according to Aspect 2 is compatible with the progressive reconstruction of Aspect 3, wherein the progressive reconstruction of Aspect 3 does not necessarily require the residual reconstruction, i.e., the progressive reconstruction is applicable without residual reconstruction.

Analogously, the compressive transmission from and to the ultrasonic head may be used in combination with the apparatuses according to Aspects 1 to 3 or in combination with a conventional measurement chain.

With respect to Aspect 2 - residual reconstruction using prior knowledge on the object being scanned - it should be noted that the prior knowledge can be, e.g.,

- one or multiple measurements of a reference object

- one or multiple measurements of the same object at one or multiple earlier time points

- a reconstructed image of a reference object or a plurality of reconstructed images from a set of reference objects

- an image of a reference object obtained from CAD data, which may also include tolerances, e.g., in form of an uncertainty associated to individual regions in the image

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus. Some or all of the method steps may be executed by (or using) a hardware apparatus, like for example, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a digital storage medium, for example a floppy disk, a DVD, a Blu-Ray, a CD, a ROM, a PROM, an EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may for example be stored on a machine readable carrier.

Other embodiments comprise the computer program for performing one of the methods described herein, stored on a machine readable carrier.

in other words, an embodiment of the inventive method is, therefore, a computer program having a program code for performing one of the methods described herein, when the computer program runs on a computer.

A further embodiment of the inventive methods is, therefore, a data carrier (or a digital storage medium, or a computer-readable medium) comprising, recorded thereon, the computer program for performing one of the methods described herein. The data carrier, the digital storage medium or the recorded medium are typically tangible and/or non-transitionary.

A further embodiment of the inventive method is, therefore, a data stream or a sequence of signals representing the computer program for performing one of the methods described herein. The data stream or the sequence of signals may for example be configured to be transferred via a data communication connection, for example via the Internet.

A further embodiment comprises a processing means, for example a computer, or a programmable logic device, configured to or adapted to perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

Definitions used for the description of the above aspects will be given below:

| Object | This is the physical being scanned. |
|---|---|
| Excitation signals | These are the signals being played back by the ultrasonic transducers. K transducers are used to transmit (Tx), N transducers are used to receive. If the same transducers are used both for Tx and Rx, then K = $N$.<br><br>The excitation signal is denoted by the matrix A. The size of the matrix is the number of the time samples x number of active Tx transducers. |
| Image | One-dimensional (1D), two-dimensional (2D), or three-dimensional (3D) sampled representation of a physically existing object such that each element (e.g., a pixel or a voxel) represents some physically interpretable feature of the object, such as a local absorption coefficient, or a boundary between regions with different impedances. |
| Reconstruction | This is the process of recovering the image out of the ultrasonic measurements. The estimate for the image is termed reconstructed image. |
| Reconstructed image | $\tilde{f}$ is the image resulting from the reconstruction process |
| Reference image | $f_{ref}$ is an image of reference object.<br><br>$\tilde{f}_{ref}$ is a rotated version of the reference image. |

(continued)

| Residual image | $\Delta f$ is the image once a *priori* information has been removed. In the simplest embodiment, the residual image is obtained from the image once a reference image has been subtracted. |
|---|---|
| Measurement, measurement run | A measurement is the collection of signals measured at the receiver transducers once we excite the object with excitation signals. Let $x_n$ denote a vector containing T temporal coefficients sampled from the analog receive signal at the n-th receiver transducer. Let further X donate a matrix of size T x N, such that the n-th column is $x_n$. Subsequent measurements without moving the transducers (but potentially with different excitation signals $A_m$) are donated by $X_m$, where m is the index of the measurement run. A total number of M measurement runs is done. |

References

[1] Aras Azimipanah. Compressive sensing based non-destructive testing using ultrasonic arrays. Master thesis, University of Ontario Institute of Technology, 2013. URL: https://ir.library.dc-uoit.ca/bitstream/10155/351/1/Azimipanah_Aras.pdf.

[2] Aras Azimipanah and Shahram Shahbaz Panahi. Experimental results of compressive sensing based imaging in ultrasonic non-destructive testing. In IEEE 5th International Workshop on Computational Advances in Multi-Sensor Adaptive Processing (CAMSAP), pages 336-339, St. Martin, December 2013. doi:10.1109/CAMSAP.2013.6714076.

[3] Bao Binghao, Geng Wei, Lu Xin, Pan Haibin, Song Shoupeng, Xu Baowen, and Zhao Tengfei. Method using ultrasound for the non-destructive examination of a test body, March 26 2014. CN Patent App. CN 201,310,681,384. URL: http://www.google.com/patents/CN103679762A?cl=en.

[4] Florian Boßmann, Gerlind Plonka, Thomas Peter, Oliver Nemitz, and Till Schmitte. Sparse deconvolution methods for ultrasonic NDT. Journal of Nondestructive Evaluation, 31(3):225-244, 2012. doi:10.1007/s10921-012-0138-8.

[5] Emmanuel J. Candès, Justin K. Romberg, and Terence Tao. Robust uncertainty principles: exact signal reconstruction from highly incomplete frequency information. IEEE Transactions on information Theory, 52(2):489-509, February 2006. doi:10.1109/TIT.2005.862083.

[6] Emmanuel J. Candès and Terence Tao. Near-optimal signal recovery from random projections: Universal encoding strategies? IEEE Transactions on Information Theory, 52(12):5406-5425, December 2006. doi:10.1109/TIT.2006.885507.

[7] David L. Donoho. Compressed sensing. IEEE Transactions on information Theory, 52(4):1289-1306, April 2006. doi:10.1109/TIT.2006.871582.

[8] Yonina C. Eldar and Ewa Matusiak. Sub-nyquist sampling of short pulses, September 16 2014. US Patent 8,836,557. URL: http://www.google.com/patents/US8836557.

[9] Christian Kexel and Jochen Moll. Deconvolution processing for improved acoustic wavefield imaging. Case Studies in Nondestructive Testing and Evaluation, 2:77-83, October 2014. doi:10.1016/j.csndt.2014.10.002.

[10] Michael Kr6ning, Dieter Hentschel, Ludwig von Bernus, Andrey Bulavinov, and Krishna Reddy. Method using ultrasound for the non-destructive examination of a test body, February 5 2013. US Patent 8,365,600. URL: http://www.google.com/patents/US8365600.

[11] Silvio E. Kruger, Guy Lamouche, Daniel Lévesque, and Jean-Pierre Monchalin. Method and system for determining material properties using ultrasonic attenuation, April 8 2008. US Patent 7,353,709. URL: http://www.google.co.in/patents/US7353709.

[12] Hervé Liebgott, Adrian Basarab, Denis Kouame, Olivier Bernard, and Denis Friboulet. Compressive sensing in medical ultrasound. In IEEE International Ultrasonics Symposium (IUS), pages 1-6, Dresden, October 2012. doi:10.1109/ULTSYM.2012.0486.

[13] Alessandro Perelli, Tommaso Di Ianni, Alessandro Marzani, Luca De Marchi, and Guido Masetti. Model-based compressive sensing for damage localization in lamb wave inspection. IEEE Transactions on Ultrasonics, Ferroelectrics, and Frequency Control, 60(10):2089- 2097, October 2013. doi:10.1109/TUFFC.2013.2799.

[14] Marco Ricci, Sergio Callegari, Salvatore Caporale, Marcello Monticelli, Luigi Battaglini, Massimiliano Eroli, Luca Senni, Riccardo Rovatti, Gianluca Setti, and Pietro Burrascano. Exploiting non-linear chirp and sparse deconvolution to enhance the performance of pulsecompression ultrasonic NDT. In IEEE International Ultrasonics Symposium (IUS), pages 1489-1492, October 2012. doi:10.1109/ULTSYM.2012.0372.

[15] Charles Soussen, Jérôme Idier, Ewen Carcreff, Laurent Simon, and Catherine Potel. Ultrasonic non destructive testing based on sparse deconvolution. In 10th Anglo-French Physical Acoustics Conference (AFPAC), pages 336-339, 2011. doi:10.1088/1742-6596/353/1/012018.

[16] Ronen Tur, Yonina C. Eldar, and Zvi Friedman. Innovation rate sampling of pulse streams with application to ultrasound imaging. IEEE Transactions on Signal Processing, 59(4):1827-1842, April 2011. doi:10.1109/TSP.2011.2105480.

[17] Noam Wagner, Yonina C. Eldar, Arie Feuer, and Gilad Daninand Zvi Friedman. Xampling in ultrasound imaging. In SPIE Medical imaging Conference, pages 1-17, Orlando Florida, 2011. doi:10.1117/12.877818.

[18] Guang-Ming Zhang, David M. Harvey, and Derek R. Braden. Advanced acoustic microimaging using sparse signal representation for the evaluation of microelectronic packages. IEEE Transactions on Advanced Packaging, 29(2):271-283, May 2006. doi:10.1109/TADVP.2005.853553.

[19] Guang-Ming Zhang, David M. Harvey, and Derek R. Braden. Effect of sparse basis selection on ultrasonic signal representation. Ultrasonics, 45(1-4):82-91, December 2006. doi:10.1016/j.ultras.2006.07.005.

[20] Guang-Ming Zhang, David M. Harvey, and Derek R. Braden. Sparse deconvolution of ultrasonic NDE traces - a preliminary study. In Ultrasonics Symposium, 2008. IUS 2008. IEEE, pages 176-179, November 2008. doi:10.1109/ULTSYM.2008.0043.

[21] Guang-Ming Zhang, Cheng-Zhong Zhang, and David M. Harvey. Sparse signal representation and its applications in ultrasonic NDE. Ultrasonics, 52(3):351-363, March 2012. doi:10.1016/j.ultras.2011.10.001.

## Claims

1. A method (2000) for reconstructing an image of an object based on received signals and prior knowledge signals using ultrasonic equipment, the method comprising:

removing (2002) information within the received signals, that are contained by the received signals and the prior knowledge signals to obtain a residual signal $\Delta X$; and
reconstructing (2004) an image $\Delta f$ of the object based on the residual signal wherein the reconstructing is performed based on the following formula:

$$\arg \min_{\Delta f} g(\Delta X, \text{ForwardModel}(A, \Delta f)) + \lambda \cdot h(\Delta f),$$

wherein Forward Model ($A, \Delta f$) is a function that simulates the measured signal we would obtain if we excited a volume described by the image $\Delta f$ with the excitation signal described by A, the function $g(X_1, X_2) \in \mathbb{R}$

quantifies the difference between the two measured signals $X_1$ and $X_2$, and the function $h(\Delta f) \in \mathbb{R}^+$ quantifies the sparsity of the residual image $\Delta f$;

wherein prior knowledge comprises signals belonging to one or more measurements of a reference object, signals belonging to one or more measurements of the object to be reconstructed and serving as reference, to one or more images of a reference object, to one or more images of a set of reference objects and/or to one or more simulated images of a reference object.

2. The method (2000) according to claim 1, further comprising the step of synthetizing a virtual reference object based on a reference object misaligned with the object to be reconstructed, before removing the information, wherein the synthetizing is performed taking into account the misalignment.

3. The method (2000) according to one of claims 1-2, further comprising the step of aligning the reference object misaligned with the object to be reconstructed to obtain the difference between the misaligned reference object and an aligned reference object; and
performing the step of reconstructing (2004) the image based on the residual signal and the difference between the misaligned reference object and the aligned reference object.

4. The method (2000) according to claim 3, wherein the reconstructing is performed using the formula:

$$\arg \min_{\Delta f} g(\boldsymbol{\Delta X}, \text{ForwardModel}(\boldsymbol{A}, \Delta \boldsymbol{f} + \boldsymbol{f}_{\text{mis}})) + \lambda \cdot h(\boldsymbol{\Delta f}),$$

wherein ForwardModel $(\boldsymbol{A}, \Delta \boldsymbol{f})$ is a function that simulates the measured signal we would obtain if we excited a volume described by the image $\Delta \boldsymbol{f}$ with the excitation signal described by A, the function $g(\boldsymbol{X}_1, \boldsymbol{X}_2) \in \mathbb{R}$ quantifies the difference between the two measured signals $\boldsymbol{X}_1$ and $\boldsymbol{X}_2$, and the function $h(\boldsymbol{\Delta f}) \in \mathbb{R}^+$ quantifies the sparsity of the residual image $\Delta \boldsymbol{f}$, and wherein the signal $\boldsymbol{f}_{\text{mis}}$ is additionally contained in the data fidelity term to correct difference between the misaligned reference object and the aligned reference object.

5. The method (2000) according to one of claims 1 to 4 further comprising the steps of the following method for performing multiple measurements of an object, this method comprising:

providing (1002) a plurality of non-focused excitation signals to a volume comprising the object in accordance to excitation properties;
receiving (1004) a plurality of response signals resulting from reflections of the non-focused excitation signals at the object and/or within the volume; and
reconstructing (1006) an image of the object based on the received signals;
analyzing the reconstructed image to output an information; and
adapting the excitation properties for the next measurement based on the information of the analyzing step ;
wherein providing the plurality of non-focused signals comprises the substep of generating ultrasonic waves such that the ultrasonic energy is not concentrated in a single point or towards a single direction or within a confined volume within the object and/or volume.

6. The method (2000) according to one of claims 1 to 5 further comprising the steps of the following method for performing multiple measurements of an object to be reconstructed using ultrasonic equipment, the method comprising:

performing (3002) a first measurement comprising the step of providing a plurality of excitation signals to a volume comprising the object and the step of receiving a plurality of response signals resulting from reflections of the excitation signal at the object and/or within the volume;
starting (3006) to reconstruct an image of the object based on the received signals of the first measurement;
performing (3004) a second measurement comprising the step of providing a plurality of excitation signals to the volume and the step of receiving a plurality of response signals resulting from reflections of the excitation signals at the object and/or within the volume; and
continuing (3008) to reconstruct the image of the object based on the received signals of the first and second measurement,
wherein the step of starting to reconstruct is in parallel to the step of performing the second measurement;
continuing (3008) to reconstruct comprises i reconstructions, wherein the i-th reconstruction makes use of the intermediate results of the previous one, i - 1, comprising the following substeps:

when the excitation signals are the same, $A_i = A_{i-1}$, updating a cost function with the measurement matrix being equal to the weighted sum of the previous measurements; and
when the excitation signals differ, $A_i \neq A_{i-1}$, solving a different optimization problem than the one of the (i-1)-th reconstruction.

7. An apparatus for reconstructing an image of an object based on received signals and prior knowledge signals using ultrasonic equipment, the apparatus comprising:

a processor (106, 107) configured to remove information within the received signals, that are contained by the received signals and the prior knowledge signals to obtain a residual signal $\Delta X$, and configured to reconstruct the image $\Delta f$ based on the residual signal;
wherein the processor (103) is configured to perform the reconstructing based on the following formula:

$$\arg \min_{\Delta f} g(\Delta X, \text{ForwardModel}(A, \Delta f)) + \lambda \cdot h(\Delta f),$$

wherein Forward Model $(A, \Delta f)$ is a function that simulates the measured signal we would obtain if we excited a volume described by the image $\Delta f$ with the excitation signal described by $A$, the function $g(X_1, X_2) \in \mathbb{R}$ quantifies the difference between the two measured signals $X_1$ and $X_2$, and the function $h(\Delta f) \in \mathbb{R}^+$ quantifies the sparsity of the residual image $\Delta f$;
wherein prior knowledge comprises signals belonging to one or more measurements of a reference object, signals belonging to one or more measurements of the object to be reconstructed and serving as reference, to one or more images of a reference object, to one or more images of a set of reference objects and/or to one or more simulated images of a reference object.

8. Computer readable digital storage medium having stored thereon a computer program having a program code for performing, when running on a computer, a method according to one of the claims 1 to 6.

**Patentansprüche**

1. Ein Verfahren (2000) zum Rekonstruieren eines Bildes eines Objektes auf der Basis eines empfangenen Signals und Vorabwissen-Signalen unter Verwendung einer Ultraschallausrüstung, wobei das Verfahren folgende Schritte aufweist:

Entfernen (2002) von Informationen innerhalb der empfangenen Signale, die in den empfangenen Signalen und den Vorabwissen-Signalen enthalten sind, um ein Restsignal $\Delta x$ zu erhalten; und
Rekonstruieren (2004) eines Bildes $\Delta f$ des Objektes auf der Basis des Restsignals, wobei das Rekonstruieren auf der Basis der folgenden Formel ausgeführt wird:

$$\arg \min_{\Delta f} g(\Delta x, \text{ForwardModel}(A, \Delta f)) + \lambda \cdot h(\Delta f),$$

wobei ForwardModel $(A, \Delta f)$ eine Funktion ist, die das gemessene Signal simuliert, welches erhalten werden würde, wenn ein durch das Bild $\Delta f$ beschriebenes Volumen mit einem durch A beschriebenen Anregungssignal angeregt werden würde, wobei die Funktion $g(X_1, X_2) \in \mathbb{R}$ die Differenz zwischen den zwei gemessenen Signalen $X_1$ und $X_2$ quantifiziert, und die Funktion $h(\Delta f) \in \mathbb{R}$ die Spärlichkeit des Restbildes $\Delta f$ quantifiziert;
wobei das Vorabwissen Signale, die zu einer oder mehreren Messungen eines Referenzobjektes gehören, Signale, die zu einer oder mehreren Messungen des zu rekonstruierenden Objektes gehören und als Referenz dienen, zu einem oder mehreren Bildern eines Referenzobjektes, zu einem oder mehreren Bildern einer Menge an Referenzobjekten gehören und/oder zu einem oder mehreren simulierten Bildern eines Referenzobjektes gehören, aufweist.

2. Das Verfahren (2000) gemäß Anspruch 1, das ferner den Schritt eines Synthetisierens eines virtuellen Referenzobjektes auf der Basis eines Referenzobjektes aufweist, welches in Bezug auf das zu rekonstruierende Objekt fehlangepasst ist, vor dem Entfernen der Informationen, wobei das Synthetisieren unter Berücksichtigung der Fehlanpassung ausgeführt wird.

3. Das Verfahren (2000) gemäß einem der Ansprüche 1 bis 2, das ferner den Schritt eines Ausrichtens des Referenzobjektes, welches hinsichtlich des zu rekonstruierenden Objektes fehlausgerichtet ist, um die Differenz zwischen dem fehlausgerich-teten Referenzobjekt und einem ausgerichteten Referenzobjekt zu erhalten; und eines Ausführen des Schrittes des Rekonstruierens (2004) des Bildes auf der Basis des Restsignals und der Differenz zwischen dem fehlausgerichteten Referenzobjekt und dem ausgerichteten Referenzobjekt aufweist.

4. Das Verfahren (2000) gemäß Anspruch 3, wobei das Rekonstruieren unter Verwendung der folgenden Formel ausgeführt wird:

$$\arg \min_{\Delta f} g(\Delta X, \text{ForwardModel}(A, \Delta f + f_{mis})) + \lambda \cdot h(\Delta f),$$

wobei ForwardModel ($A, \Delta f$) eine Funktion ist, die das gemessene Signal simuliert, das erhalten werden würde, wenn ein durch das Bild $\Delta f$ beschriebenes Volumen mit einem durch A beschriebenen Anregungssignal angeregt werden würde, wobei die Funktion $g(X_1, X_2) \in \mathbb{R}$ die Differenz zwischen den zwei gemessenen Signalen $X_1$ und $X_2$ quantifiziert, und die Funktion $h(\Delta f) \in \mathbb{R}$ die Spärlichkeit des Restbildes $\Delta f$ quantifiziert, wobei das Signal $f_{mis}$ zusätzlich in dem Datentreueterm enthalten ist, um eine Differenz zwischen dem fehlausgerichteten Referenzobjekt und dem ausgerichteten Referenzobjekt zu korrigieren.

5. Das Verfahren (2000) gemäß einem der Ansprüche 1 bis 4, das ferner die Schritte des folgenden Verfahrens zum Ausführen mehrerer Messungen eines Objektes aufweist, wobei dieses Verfahren folgende Schritte aufweist:

Bereitstellen (1002) einer Mehrzahl von nichtfokussierten Anregungssignalen an ein Volumen, das das Objekt aufweist, gemäß Anregungseigenschaften;
Empfangen (1004) einer Mehrzahl von Antwortsignalen, die aus Reflexionen der nichtfokussierten Anregungssignale an dem Objekt und/oder innerhalb des Volumens resultieren; und
Rekonstruieren (1006) eines Bildes des Objektes auf der Basis der empfangenen Signale;
Analysieren des rekonstruierten Bildes, um eine Information auszugeben; und
Anpassen der Anregungseigenschaften für die nächste Messung auf der Basis der Informationen des Analyseschrittes;
wobei das Bereitstellen der Mehrzahl von nichtfokussierten Signalen den Teilschritt eines Erzeugens von Ultraschallwellen derart aufweist, dass die Ultraschallenergie nicht in einem einzelnen Punkt oder hin zu einer einzelnen Richtung oder innerhalb eines begrenzten Volumens innerhalb des Objektes und/oder Volumens konzentriert ist.

6. Das Verfahren (2000) gemäß einem der Ansprüche 1 bis 5, das ferner die Schritte des folgenden Verfahrens zum Ausführen mehrerer Messungen eines zu rekonstruierenden Objektes unter Verwendung einer Ultraschalleinrichtung aufweist, wobei das Verfahren folgende Schritte aufweist:

Ausführen (3002) einer ersten Messung, die einen Schritt eines Bereitstellens einer Mehrzahl von Anregungssignalen an ein Volumen, das das Objekt aufweist, und den Schritt eines Empfangens einer Mehrzahl von Antwortsignalen, die aus Reflexionen des Anregungssignals an dem Objekt und/oder innerhalb des Volumens resultieren, aufweist;
Starten (3006) mit der Rekonstruktion eines Bildes des Objektes auf der Basis der empfangenen Signale der ersten Messung;
Ausführen (3004) einer zweiten Messung, die den Schritt eines Bereitstellens einer Mehrzahl von Anregungssignalen an das Volumen und den Schritt eines Empfangens einer Mehrzahl von Antwortsignalen, die aus Reflexionen der Anregungssignale an dem Objekt und/oder innerhalb des Volumens resultieren, aufweist; und

Fortsetzen (3008) mit der Rekonstruktion des Bildes des Objektes auf der Basis der empfangenen Signale der ersten und der zweiten Messung,
wobei der Schritt des Startens mit der Rekonstruktion parallel zu dem Schritt des Ausführens der zweiten Messung ist;
wobei das Fortsetzen (3008) mit der Rekonstruktion i Rekonstruktionen aufweist,
wobei die i-te Rekonstruktion die Zwischenergebnisse der vorherigen nutzt, i-1, wobei dies die folgenden Teilschritte aufweist:

wenn die Anregungssignale gleich sind, A; = $A_{i-1}$, Aktualisieren einer Kostenfunktion, wobei die Messmatrix gleich der gewichteten Summe der vorherigen Messungen ist; und
wenn die Anregungssignale sich unterscheiden, $A_i \neq A_{i-1}$, Lösen eines anderen Optimierungsproblems als das der (i-1)-ten Rekonstruktion.

**7.** Eine Vorrichtung zum Rekonstruieren eines Bildes eines Objektes auf der Basis von Basis von empfangenen Signalen und Vorabwissen-Signalen unter Verwendung einer Ultraschallausrüstung, wobei die Vorrichtung folgende Merkmale aufweist:

einen Prozessor (106, 107), der dazu konfiguriert ist, Informationen innerhalb der empfangenen Signale, die in den empfangenen Signalen und den Vorabwissen-Signalen enthalten sind, zu entfernen, um ein Restsignal $\Delta x$ zu erhalten, und dazu konfiguriert ist, das Bild $\Delta f$ auf der Basis des Restsignals zu rekonstruieren,
wobei der Prozessor (103) dazu konfiguriert ist, das Rekonstruieren auf der Basis der folgenden Formel ausgeführt wird:

$$\arg \min_{\Delta f} g(\Delta x, \text{ForwardModel}(A, \Delta f)) + \lambda \cdot h(\Delta f),$$

wobei ForwardModel (*A,Δf*) eine Funktion ist, die das gemessene Signal simuliert, welches erhalten werden würde, wenn ein durch das Bild $\Delta f$ beschriebenes Volumen mit einem durch A beschriebenen Anregungssignal angeregt werden würde, wobei die Funktion $g(X_1, X_2) \in \mathbb{R}$ die Differenz zwischen den zwei gemessenen Signalen $X_1$ und $X_2$ quantifiziert, und die Funktion $h(\Delta f) \in \mathbb{R}$ die Spärlichkeit des Restbildes $\Delta f$ quantifiziert;
wobei das Vorabwissen Signale, die zu einer oder mehreren Messungen eines Referenzobjektes gehören, Signale, die zu einer oder mehreren Messungen des zu rekonstruierenden Objektes gehören und als Referenz dienen, zu einem oder mehreren Bildern eines Referenzobjektes, zu einem oder mehreren Bildern einer Menge an Referenzobjekten gehören und/oder zu einem oder mehreren simulierten Bildern eines Referenzobjektes gehören, aufweist.

**8.** Ein computerlesbares digitales Speichermedium, auf dem ein Computerprogramm mit einem Programmcode gespeichert ist, zum Ausführen, wenn dasselbe auf einem Computer abläuft, eines Verfahrens gemäß einem der Ansprüche 1 bis 6.

**Revendications**

**1.** Procédé (2000) pour reconstruire une image d'un objet sur base de signaux reçus et de signaux de connaissance antérieure à l'aide d'un équipement ultrasonique, le procédé comprenant le fait de:

éliminer (2002) les informations dans les signaux reçus qui sont contenues par les signaux reçus et les signaux de connaissance antérieure pour obtenir un signal résiduel *ΔX;* et
reconstruire (2004) une image *Δf* de l'objet sur base du signal résiduel,
dans lequel la reconstruction est effectuée sur base de la formule suivante:

$$arg\ min_{\Delta f}\ g(\Delta X, \text{ForwardModel}(A, \Delta f)) + \lambda \cdot h(\Delta f),$$

où Forward Model (*A,Δf*) est une fonction qui simule le signal mesuré que nous obtiendrions si nous excitions un volume décrit par l'image $\varDelta f$ par le signal d'excitation décrit par $\boldsymbol{A}$, la fonction $g(X_1, X_2) \in \mathbb{R}$ quantifie la différence entre les deux signaux mesurés $\boldsymbol{X}_1$ et $\boldsymbol{X}_2$, et la fonction $h(\varDelta f) \in \mathbb{R}^+$ quantifie le clairsemé de l'image résiduelle $\varDelta \boldsymbol{f}$;

dans lequel la connaissance antérieure comprend les signaux appartenant à une ou plusieurs mesures d'un objet de référence, les signaux appartenant à une ou plusieurs mesures de l'objet à reconstruire et servant de référence, à une ou plusieurs images d'un objet de référence, à une ou plusieurs images d'un ensemble d'objets de référence et/ou à une ou plusieurs images simulées d'un objet de référence.

2. Procédé (2000) selon la revendication 1, comprenant, par ailleurs l'étape consistant à synthétiser un objet de référence virtuel sur base d'un objet de référence en désalignement, avec l'objet à reconstruire, avant d'éliminer les informations, dans lequel la synthétisation est effectuée en tenant compte du désalignement.

3. Procédé (2000) selon l'une des revendications 1 à 2, comprenant par ailleurs l'étape consistant à aligner l'objet de référence en désalignement avec l'objet à reconstruire pour obtenir la différence entre l'objet de référence en désalignement et un objet de référence aligné; et

effectuer l'étape consistant à reconstruire (2004) l'image sur base du signal résiduel et de la différence entre l'objet de référence en désalignement et l'objet de référence aligné.

4. Procédé (2000) selon la revendication 3, dans lequel la reconstruction est effectuée selon la formule:

$$arg\, min_{\varDelta f}\ g(\varDelta X, \text{ForwardModel}(A, \varDelta f + f_{mis}))\ +\ \lambda \cdot h(\varDelta f),$$

où ForwardModel $(\boldsymbol{A}, \varDelta \boldsymbol{f})$ est une fonction qui simule le signal mesuré que nous obtiendrions si nous excitions un volume décrit par l'image $\varDelta \boldsymbol{f}$ par le signal d'excitation décrit par $\boldsymbol{A}$, la fonction $g(X_1, X_2) \in \mathbb{R}$ quantifie la différence entre les deux signaux mesurés $\boldsymbol{X}_1$ et $\boldsymbol{X}_2$, et la fonction $h(\varDelta f) \in \mathbb{R}^+$ quantifie le clairsemé de l'image résiduelle $\varDelta \boldsymbol{f}$, et où le signal $\boldsymbol{f}_{mis}$ est par ailleurs contenu dans le terme de fidélité des données pour corriger la différence entre le l'objet de référence en désalignement et l'objet de référence aligné.

5. Procédé (2000) selon l'une des revendications 1 à 4, comprenant par ailleurs les étapes du procédé suivant pour effectuer des mesures multiples d'un objet, ce procédé comprenant le fait de:

fournir (1002) une pluralité de signaux d'excitation non focalisés à un volume comprenant l'objet en conformité avec les propriétés d'excitation;
recevoir (1004) une pluralité de signaux de réponse résultant de réflexions des signaux d'excitation non focalisés au niveau de l'objet et/ou dans le volume; et
reconstruire (1006) une image de l'objet sur base des signaux reçus;
analyser l'image reconstruite pour sortir une information; et
adapter les propriétés d'excitation pour la mesure suivante sur base des informations de l'étape d'analyse;
dans lequel la fourniture de la pluralité de signaux non focalisés comprend la sous-étape consistant à générer des ondes ultrasonores de sorte que l'énergie ultrasonore ne soit pas concentrée en un seul point ou vers une seule direction ou dans un volume confiné dans l'objet et/ou dans le volume.

6. Procédé (2000) selon l'une des revendications 1 à 5, comprenant par ailleurs les étapes du procédé suivant pour effectuer des mesures multiples d'un objet à reconstruire à l'aide d'un équipement ultrasonique, le procédé comprenant le fait de:

effectuer (3002) une première mesure comprenant l'étape consistant à fournir une pluralité de signaux d'excitation à un volume comprenant l'objet et l'étape consistant à recevoir une pluralité de signaux de réponse résultant de réflexions du signal d'excitation au niveau de l'objet et/ou dans le volume;
commencer (3006) à reconstruire une image de l'objet sur base des signaux reçus de la première mesure;
effectuer (3004) une deuxième mesure comprenant l'étape consistant à fournir une pluralité de signaux d'excitation au volume et l'étape consistant à recevoir une pluralité de signaux de réponse résultant de réflexions des signaux d'excitation au niveau de l'objet et/ou dans le volume; et continuer (3008) à reconstruire l'image de l'objet sur base des signaux reçus de la première et de la deuxième mesure, dans lequel l'étape consistant à commencer la reconstruction est parallèle à l'étape consistant à effectuer la deuxième mesure; la continuation (3008) de la reconstruction comprend i reconstructions, où la i-ème reconstruction utilise les résultats intermédiaires de la précédente, i-1, comprenant les sous-étapes suivantes consistant à:

lorsque les signaux d'excitation sont les mêmes, $A_i = A_{i-1}$, mettre à jour une fonction de coût, la matrice de mesure étant égale à la somme pondérée des mesures précédentes; et

lorsque les signaux d'excitation diffèrent, $A_i \neq A_{i-1}$, résoudre un problème d'optimisation différent de celui de la (i-1)-ème reconstruction.

7. Appareil pour reconstruire une image d'un objet sur base de signaux reçus et de signaux de connaissance antérieure à l'aide d'un équipement ultrasonique, l'appareil comprenant: un processeur (106, 107) configuré pour éliminer les informations dans les signaux reçus qui sont contenues par les signaux reçus et les signaux de connaissance antérieure pour obtenir un signal résiduel $\Delta X$, et configuré pour reconstruire l'image $\Delta f$ sur base du signal résiduel;

dans lequel le processeur (103) est configuré pour effectuer la reconstruction sur base de la formule suivante:

$$arg\ min_{\Delta f}\ g(\Delta X, \mathrm{ForwardModel}(A, \Delta f)) + \lambda \cdot h(\Delta f),$$

où Forward Model ($A$, $\Delta f$)est une fonction qui simule le signal mesuré que nous obtiendrions si nous excitions un volume décrit par l'image $\Delta f$ par le signal d'excitation décrit par $A$, la fonction $g(X_1, X_2) \in \mathbb{R}$ quantifie

la différence entre les deux signaux mesurés $X_1$ et $X_2$, et la fonction $h(\Delta f) \in \mathbb{R}^+$ quantifie le clairsemé de l'image résiduelle $\Delta f$,

dans lequel la connaissance antérieure comprend les signaux appartenant à une ou plusieurs mesures d'un objet de référence, les signaux appartenant à une ou plusieurs mesures de l'objet à reconstruire et servant de référence, à une ou plusieurs images d'un objet de référence, à une ou plusieurs images d'un ensemble d'objets de référence et/ou à une ou plusieurs images simulées d'un objet de référence.

8. Support de mémoire numérique lisible par ordinateur présentant, y mémorisé, un programme d'ordinateur présentant un code de programme pour réaliser, lorsqu'il est exécuté sur un ordinateur, un procédé selon l'une des revendications 1 à 6.

<u>1000</u>

FIG 1A

FIG 1B

FIG 1C

2000

## FIG 2A

## FIG 2B

118 ⎯ $X_{ref}$

A

121

generate
reference
measurements ⎯ 108

119 ⎯ $f_{ref}$

## FIG 2C

118 ⎯ $X_{ref}$

A

121

measure ⎯ 102

## FIG 2D

EP 3 338 082 B1

FIG 2E

FIG 2F

EP 3 338 082 B1

FIG 2G

3000

3002

3006

3004

3008

FIG 3A

FIG 3B

FIG 3C

EP 3 338 082 B1

401    404    402    405    403

| PC | ↔ | external unit | ↔ | ultrasonic head |

FIG 4A

EP 3 338 082 B1

ultrasonic head

403

411A

411N

407a

407b

407c

407n

409

combining network

407

421

42H

405

408

FIG 4B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2010064811 A1
- US 2013253325 A1
- WO 2015140177 A
- GB 2192120 A
- KR 20080102785
- DE 3321269 A1

- CN 201310
- VN 681384
- US 8836557 B
- US 8365600 B
- US 7353709 B

### Non-patent literature cited in the description

- Compressive sensing based non-destructive testing using ultrasonic arrays. **ARAS AZIMIPANAH.** Master thesis. University of Ontario Institute of Technology, 2013
- **ARAS AZIMIPANAH ; SHAHRAM SHAHBAZ PANAHI.** Experimental results of compressive sensing based imaging in ultrasonic non-destructive testing. *IEEE 5th International Workshop on Computational Advances in Multi-Sensor Adaptive Processing (CAMSAP),* December 2013, 336-339
- **BAO BINGHAO ; GENG WEI ; LU XIN ; PAN HAIBIN ; SONG SHOUPENG ; XU BAOWEN ; ZHAO TENGFEI.** *Method using ultrasound for the non-destructive examination of a test body,* 26 March 2014
- **FLORIAN BOßMANN ; GERLIND PLONKA ; THOMAS PETER ; OLIVER NEMITZ ; TILL SCHMITTE.** Sparse deconvolution methods for ultrasonic NDT. *Journal of Nondestructive Evaluation,* 2012, vol. 31 (3), 225-244
- **EMMANUEL J. CANDÈS ; JUSTIN K. ROMBERG ; TERENCE TAO.** Robust uncertainty principles: exact signal reconstruction from highly incomplete frequency information. *IEEE Transactions on information Theory,* February 2006, vol. 52 (2), 489-509
- **EMMANUEL J. CANDÈS ; TERENCE TAO.** Near-optimal signal recovery from random projections: Universal encoding strategies?. *IEEE Transactions on Information Theory,* December 2006, vol. 52 (12), 5406-5425
- **DAVID L. DONOHO.** Compressed sensing. *IEEE Transactions on information Theory,* April 2006, vol. 52 (4), 1289-1306
- **YONINA C. ELDAR ; EWA MATUSIAK.** *Sub-nyquist sampling of short pulses,* 16 September 2014
- **CHRISTIAN KEXEL ; JOCHEN MOLL.** Deconvolution processing for improved acoustic wavefield imaging. *Case Studies in Nondestructive Testing and Evaluation,* October 2014, vol. 2, 77-83

- **MICHAEL KR6NING ; DIETER HENTSCHEL ; LUDWIG VON BERNUS ; ANDREY BULAVINOV ; KRISHNA REDDY.** *Method using ultrasound for the non-destructive examination of a test body,* 05 February 2013
- **SILVIO E. KRUGER ; GUY LAMOUCHE ; DANIEL LÉVESQUE ; JEAN-PIERRE MONCHALIN.** *Method and system for determining material properties using ultrasonic attenuation,* 08 April 2008
- **HERVÉ LIEBGOTT ; ADRIAN BASARAB ; DENIS KOUAME ; OLIVIER BERNARD ; DENIS FRIBOULET.** Compressive sensing in medical ultrasound. *IEEE International Ultrasonics Symposium (IUS),* October 2012, 1-6
- **ALESSANDRO PERELLI ; TOMMASO DI LANNI ; ALESSANDRO MARZANI ; LUCA DE MARCHI ; GUIDO MASETTI.** Model-based compressive sensing for damage localization in lamb wave inspection. *IEEE Transactions on Ultrasonics, Ferroelectrics, and Frequency Control,* October 2013, vol. 60 (10), 2089-2097
- **MARCO RICCI ; SERGIO CALLEGARI ; SALVATORE CAPORALE ; MARCELLO MONTICELLI ; LUIGI BATTAGLINI ; MASSIMILIANO EROLI ; LUCA SENNI ; RICCARDO ROVATTI ; GIANLUCA SETTI ; PIETRO BURRASCANO.** Exploiting non-linear chirp and sparse deconvolution to enhance the performance of pulsecompression ultrasonic NDT. *IEEE International Ultrasonics Symposium (IUS),* October 2012, 1489-1492
- **CHARLES SOUSSEN ; JÉRÔME IDIER ; EWEN CARCREFF ; LAURENT SIMON ; CATHERINE POTEL.** Ultrasonic non destructive testing based on sparse deconvolution. *10th Anglo-French Physical Acoustics Conference (AFPAC),* 2011, 336-339

- **RONEN TUR ; YONINA C. ELDAR ; ZVI FRIED-MAN.** Innovation rate sampling of pulse streams with application to ultrasound imaging. *IEEE Transactions on Signal Processing,* April 2011, vol. 59 (4), 1827-1842
- **NOAM WAGNER ; YONINA C. ELDAR ; ARIE FEUER ; GILAD DANINAND ZVI FRIEDMAN.** Xampling in ultrasound imaging. *SPIE Medical imaging Conference,* 2011, 1-17
- **GUANG-MING ZHANG ; DAVID M. HARVEY ; DEREK R. BRADEN.** dvanced acoustic microimaging using sparse signal representation for the evaluation of microelectronic packages. *IEEE Transactions on Advanced Packaging,* May 2006, vol. 29 (2), 271-283
- **GUANG-MING ZHANG ; DAVID M. HARVEY ; DEREK R. BRADEN.** Effect of sparse basis selection on ultrasonic signal representation. *Ultrasonics,* December 2006, vol. 45 (1-4), 82-91
- Sparse deconvolution of ultrasonic NDE traces - a preliminary study. **GUANG-MING ZHANG ; DAVID M. HARVEY ; DEREK R. BRADEN.** Ultrasonics Symposium, 2008. IUS 2008. IEEE, November 2008, 176-179
- **GUANG-MING ZHANG ; CHENG-ZHONG ZHANG ; DAVID M. HARVEY.** Sparse signal representation and its applications in ultrasonic NDE. *Ultrasonics,* March 2012, vol. 52 (3), 351-363